Europäisches Patentamt

European Patent Office

Office européen des brevets

(11) Publication number: **0 332 752**
**A1**

## (12) EUROPEAN PATENT APPLICATION

(21) Application number: 88120589.2

(51) Int. Cl.⁴: **C12Q 1/10** , **C12Q 1/54**

(22) Date of filing: 09.12.88

The title of the invention has been amended (Guidelines for Examination in the EPO, A-III, 7.3).

(30) Priority: 05.01.88 IT 1900688

(43) Date of publication of application:
20.09.89 Bulletin 89/38

(84) Designated Contracting States:
AT BE CH DE FR GB IT LI NL SE

(71) Applicant: **Giammanco, Giuseppe**
**Via Firenze 239**
**I-95128 Catania(IT)**

(72) Inventor: **Giammanco, Giuseppe**
**Via Firenze 239**
**I-95128 Catania(IT)**

(74) Representative: **Bellenghi, Mario Dr. et al**
**Ing. A. Giambrocono & C. s.r.l. Via Rosolino**
**Pilo, 19/B**
**I-20129 Milano(IT)**

(54) **Enzymatic confirmation of coliform bacteria.**

(57) A process for the confirmation, by enzymatic test, of the presence of total and faecal coliform bacteria in drinking, bathing and waste water, foods and beverages, and device for carrying out said process.

FIG.1

EP 0 332 752 A1

## PROCESS FOR THE CONFIRMATION OF COLIFORM BACTERIA IN DRINKING, BATHING AND WASTE WATER, FOODS AND BEVERAGES.

This invention is concerned with a new process for confirming, by enzymatic test, the presence of total and faecal coliform bacteria in drinking, bathing and waste waters, and with a device for carrying out said process.

One purpose of this invention is the confirmation of the presence of coliform bacteria by a simplified and rapid process of ascertainment by an enzymatic test.

Another purpose of this invention is to provide a device allowing to carry out said process within a short time and in an economically advantageous way.

Other purposes of the invention will become apparent to those skilled in the art, on the basis of the disclosure of the present patent application.

The search and numeration of coliform bacteria (colimetry) are usual laboratory procedures to ascertain water drinkability and to provide a sanitary verification of the quality of bathing and waste waters, foods and beverages.

The colimetric method by inoculation of scalar water, food suspension or beverage amounts into test tubes with liquid lactose medium according to the multiple tube method known as MPN (Most Probable Number) is being currently used since long in all water analysis laboratories. Said method is described in standard American methods (Standard Methods for the Examination of Water and Waste Water, APHA, AWWA, WPCF, Washington, 1980), and is one of the official water analysis methods of Italian Law (Ministry Decree February 15, 1983, Official Gazette March 26, 1983, No. 84, Prime Minister Decree February 8, 1985. Official Gazette May 9, 1985, No. 108).

Colimetric analysis by the MPN method consists of a first presumptive test and two confirmation tests, one for total, the other for faecal coliform bacteria. The presumptive test is a test which is considered potentially positive when there is evolution of gas from the medium contained in test tubes inoculated with the sample under examination, due to fermentation of lactose by coliform bacteria, if any, grown in a liquid lactose medium. Each test tube in which gas has evolved has to be then subjected to confirmation tests by sub-inoculation into two further tube tests, one containing e.g. "lactose-bile-brilliant green medium" , the other "EC medium", the former being incubated for 24-48 hrs at 36° C, the latter at 44.5° C.

The first confirmation test in lactose-bile-brilliant green medium has the purpose to reveal all coliform bacteria (total coliform bacteria), if any, grown in the test tubes of the presumptive test. Under the definition of "total coliform bacteria" several bacteria are included belonging to various genera of the family Enterobacteriaceae, i.e. Escherichia, Citrobacter, Klebsiella, Enterobacter, Hafnia, Serratia. They have in common the property of fermenting lactose, because they possess, among other enzymes, a beta-galactosidase.

The second confirmation test in EC medium has the purpose of revealing faecal coliform bacteria. These belong essentially to the species Escherichia coli, they ferment lactose and possess a beta-galactosidase like the other coliform bacteria, but they also possess the enzyme beta-glucuronidase which is absent in all other coliform bacteria.

The process of this invention consists in putting into evidence the enzymes beta-galactosidase and beta-glucuronidase by the use of two commercial synthetic glycosides, i.e. o-nitrophenyl-beta-D-galactopyranoside (ONPG) and p-nitrophenyl-beta-D-glucopyranosiduronic acid (PNPGU), or another useful substrate.

Each of the two glycosides is incorporated into a base nutrient medium (tryptose-agar or the like) to obtain two separate reactive culture media. Essential requirements of a suitable base medium are

(a) the property of favoring the growth of coliform bacteria and

(b) the absence of any own coloration, so that in case of positive reaction a color appearance may be observed (a yellow color if the above mentioned glycosides are used). As a mere non-limitative indication, the composition of a suitable base medium is given hereinbelow :

| Tryptose | 20 g |
|---|---|
| Glucose | 1 g |
| Sodium chloride | 5 g |
| Agar | 15 g |
| Distilled water | 1000 ml |

The two glycoside solutions are separately prepared in a phosphate buffer and sterilized by filtration.

The glycoside solutions are incorporated into the base medium previously sterilized and cooled to 55° C.

Although the concentration of the glycosides in the solution is not critical, it has been established that they may conveniently be present in a concentration of between 0.1 and 1.0 weight percent. Preferably, the ONPG concentration is somewhat higher in respect of PNPGU. It was observed, for example, that optimum results may be achieved with a final concentration of 0.35 weight percent of ONPG and 0.25 weight percent of PNPGU in the reactive medium. These concentrations, however, may be changed within broad limits without prejudice for the efficiency of the process.

The process of this invention is advantageously carried out by utilizing devices allowing the rapid execution of the colimetry. Particularly, each of the two glycoside solutions prepared as indicated above may be contained in a device formed by a container of appropriate form and size, such as, for instance, a test tube or an ampoule with one open end, into which a given amount of liquid lactose medium is charged, said medium coming from the presumptive test in which gas had evolved. The two devices are then incubated at 30-40° C for 18-24 hrs. A color appearance in the portion of both devices impregnated with reactive medium confirms the presence of faecal coliform, a color appearance only in the device with ONPG-agar confirms the presence of total coliform bacteria.

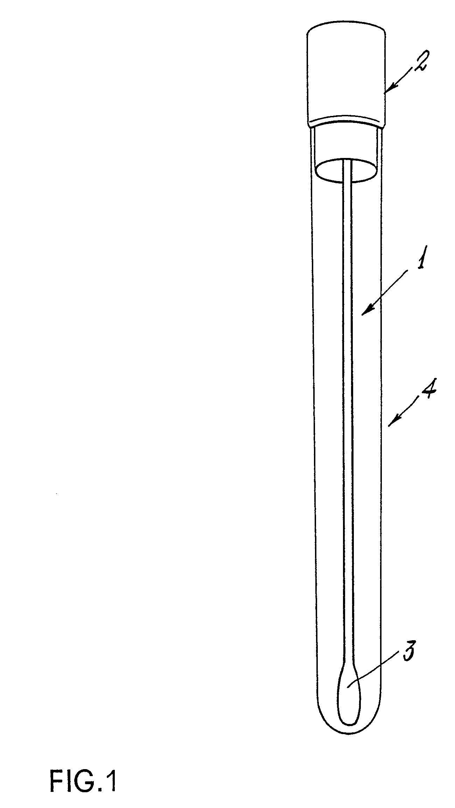

A particularly suitable device for carrying out the test is represented in Fig. 1. This device is composed by a rod (1) welded to a cap (2) by one end and provided at the other end with a support for the reactive medium. Said support may be a flat portion (3) of the rod, on which a thin layer of reactive medium is placed, or a porous portion,e.g.a small plug of cotton or blotting paper impregnated with the reactive medium. Before use, the whole device must be sterilized by physical methods, e.g. heat, UV, gamma-rays, depending on the used materials. The so prepared device is aseptically introduced in a sterile cylindric plastic or glass container (4). The whole test apparatus is composed by a couple of the above described devices, one charged with ONPG-agar, the other with PNPGU-agar, and has the purpose of confirming the presence of coliform bacteria in the medium contained in the test tube of the presumptive colimetric test, in which gas had evolved.

In order to carry out the confirmation test, each rod is extracted from the container, the end portion of the rod with the reactive medium is dipped in the liquid medium of the test tube to be confirmed, then the rod is re-introduced in the container. The two devices with ONPG-agar and PNPGU-agar are incubated at 35-37° C for 18-24 hrs. A color appearance in the portion of both devices impregnated with reactive medium confirms the presence of faecal coliform bacteria, while a color appearance in the device with ONPG-agar only confirms the presence of total coliform bacteria.

Obviously, the hereinbefore described apparatus may be utilized also when using substrates which are different from the mentioned ONPG-agar and PNPGU-agar, provided the different substrates display the same function in revealing the presence of coliform bacteria.

The advantages of the process and of the device in this invention are of an economic nature, since time and materials are saved in carrying out the confirmation tests. Actually :
- The time for the immersion of the two rods in the test tubes containing the medium in which the presence of coliform bacteria has to be confirmed (the tubes may be hundreds in number) is shorter than the time necessary when using the known system of taking with a pipet some drops from the test tubes to be confirmed and introducing them into the confirmation tubes;
- No pipets are required;
- Only a single thermostatic apparatus adjusted at 30-40° C is required instead of two thermostatic apparatuses adjusted respectively at 36° C and at 44.5° C as required by the known system;
- The incubation time is 18-24 hrs instead of 24-48 hrs required with the known system;
- The system represented in Fig. 1 may be commercially produced and distributed to the various laboratories, while the test tubes with the confirmation media according to the known system must be prepared extemporaneously in every laboratory.

Having thus described the present invention, what is intended to cover by the present patent application

EP 0 332 752 A1

is illustrated by the appended claims.

## Claims

1. A process for the confirmation of the presence of total or faecal coliform bacteria in drinking, bathing or waste water, foods and beverages in which a preliminary presumptive test has revealed the possible presence of said bacteria, said process comprising contacting two samples of medium taken from said preliminary presumptive test, separately with (a) a suitable culture medium containing a useful amount of o-nitrophenyl-beta-D-galactopyranoside and (b) a suitable culture medium containing a useful amount of p-nitrophenyl-beta-D-glucopyranosiduronic acid, and incubating said culture media at a temperature of from 30° to 40° C for 18-24 hours.

2. A process according to claim 1, wherein the useful amount of o-nitrophenil-beta-D-galactopyranoside and p-nitrophenil-beta-D-glucopyranosiduronic acid in the respective culture medium is of from 0.1 and 1.0 weight percent.

3. A process according to claims 1 and 2, wherein the useful amount of o-nitrophenil-beta-D-galactopyranoside in the culture medium is of 0.35 weight percent.

4. A process according to claims 1 and 2, wherein the useful amount of p-nitrophenil-beta-D-glucopyranosiduronic acid in the culture medium is of 0.25 weight percent.

5. A device for contacting a sample of liquid lactose medium taken from a preliminary presumptive test positive for the presence of coliform bacteria in drinking, bathing and waste waters, foods and beverages with a culture medium containing either (a) a useful amount of o-nitrophenyl-beta-D-galactopyranoside, or (b) a useful amount of p-nitrophenyl-beta-D-glucopyranosiduronic acid, or (c) a useful amount of any other suitable substrate, to confirm the presence of said coliform bacteria in said waters, said device being composed, as represented in Fig. 1, by a rod (1) welded to a cap (2) at the one end and terminating at the other end with a support, said support being a flattened portion (3) of the rod (1) or any other means suitable for taking a sample of liquid lactose medium from a preliminary presumptive test positive for the presence of coliform bacteria, said rod being adaptable, by means of the cap (2) to which it welded, to a cylindric container (4) of a suitable glass or plastic material, the length of said cylindric container being such that the flattened end (3) of the rod or the other means for taking samples causes the liquid lactose medium taken from the said preliminary positive presumptive test to come into contact with the culture medium containing the useful amount of o-nitrophenil-beta-D-galactopyranoside or p-nitrophenyl-beta-D-glucopyranosiduronic acid or any other suitable substrate.

6. An apparatus for the confirmation of the presence of total and faecal coliform bacteria in drinking, bathing and waste waters, foods and beverages said apparatus being constituted by a couple of devices as claimed in claim 5, of which one contains a solution of a useful amount of o-nitrophenyl-beta-D-galactopyranoside in a suitable culture medium, the other contains a solution of a useful amount of p-nitrophenyl-beta-D-glucopyranosiduronic acid in a suitable culture medium, or both contain other suitable substrates, to detect beta-galactosidase and beta-glucuronidase, respectively.

4

FIG.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 104, 1986, page 290, abstract no. 17436k, Columbus, Ohio, US; G. GIAMMANCO et al.: "A rapid multistrips test for determination of the enzyme profile in enterobacteria", & MICROBIOLOGICA (BOLOGNA) 1985, 8(4), 399-403 * Abstract * --- | 1 | C 12 Q 1/10<br>C 12 Q 1/54 |
| X | US-A-3 870 601 (B. WARREN) * Column 5, lines 29-53; column 6, lines 48-56 * --- | 1-3 | |
| X | ANATOMIE VAN LEEUWENHOEK, vol. 53, 1987, pages 273-277, Martinus Nijhoff Publishers, Dordrecht, NL; R.W. TREPETA et al.: "Esculinase (beta-glucosidase) for the rapid estimation of activity in bacteria utilizing a hydrolyzable substrate, p-nitrophenyl-beta-D-glucopyranoside" * Page 273, lines 5,6; page 273, lines 25,26 * --- | 1,2,4 | |
| Y | EP-A-0 163 867 (TERUMO CORP.) * Page 3, lines 9-18 * --- | 1,2 | TECHNICAL FIELDS SEARCHED (Int. Cl.4)<br><br>C 12 Q |
| Y | CHEMICAL ABSTRACTS, vol. 81, 1974, page 121, abstract no. 34262z, Columbus, Ohio, US; B.S. REDDY et al.: "Fecal bacterial beta-glucuronidase. Control by diet", & SCIENCE 1974, 183(4123), 416-17 * Abstract * --- | 1,2 | |
| X | US-A-4 184 483 (D.J. GREENSPAN) * Column 1, lines 13-22; figure 2 * --- -/- | 5,6 | |

The present search report has been drawn up for all claims

| Place of search | Date of completion of the search | Examiner |
|---|---|---|
| THE HAGUE | 18-05-1989 | VAN BOHEMEN C.G. |

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X,P | CHEMICAL ABSTRACTS, vol. 108, 1988, page 323, abstract no. 145915k, Columbus, Ohio, US; V. SKAR et al.: "Beta-glucoronidase activity related to bacterial growth in common bile duct bile in gallstone patients", & SCAND. J. GASTROENTEROL. 1988, 23(1), 83-90 * Abstract * | 4 | |
| X | CHEMICAL ABSTRACTS, vol. 101, 1984, page 335, abstract no. 107101q, Columbus, Ohio, US; J. THOMPSON et al.: "Use of a single-tube medium, o-nitrophenyl-beta-D-galactopyranoside-p henylalanine-motility sulfate, for screening of pathogenic members of the family enterobacteriaceac", & J. CLIN. MICROBIOL. 1984, 20(1), 136-7 * Abstract * | 3 | |

TECHNICAL FIELDS
SEARCHED (Int. Cl.4)

The present search report has been drawn up for all claims

| Place of search | Date of completion of the search | Examiner |
|---|---|---|
| THE HAGUE | 18-05-1989 | VAN BOHEMEN C.G. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
......................................................................
& : member of the same patent family, corresponding
   document

EPO FORM 1503 03.82 (P0401)